# EUROPEAN PATENT APPLICATION

(11) **EP 3 779 934 A1**
(43) Date of publication of application: **17.02.2021**
(21) Application number: 19757415.5
(22) Date of filing: 21.02.2019
(51) Int. Cl.: G09F 3/03, G09F 3/00, G06K 19/06, G06Q 30/06, G09F 3/02

(54) **MEDICINAL PRODUCT SALES MANAGEMENT METHOD, MEDICINAL PRODUCT SALES MANAGEMENT SYSTEM, COMPUTER PROGRAM THEREFOR, AND MEDICINAL PRODUCT INFORMATION SHEET**

(30) Priority: 22.02.2018 KR 20180020965; 03.04.2018 KR 20180038881; 21.05.2018 KR 20180057969
(71) Applicant: E Blue Co., Ltd., Gyeongsangbuk-do 39253 (KR)
(72) Inventor: LEE, Nahyeon, Ulsan 44096 (KR)
(74) Representative: Herrmann, Franz
(86) International application number: PCT/KR2019/002155
(87) International publication number: WO 2019/164308

(57) **Abstract**

The present invention relates to a medicinal product sales management method, a medicinal product sales management system, a computer program therefor, and a medicinal product information sheet. According to an aspect of the present invention, when a medicinal product, for which an order is placed by a pharmacy to a distributor, is delivered to the pharmacy and the inventory is managed, a single medicinal product information sheet, in which medicinal product price information printed in numbers, medicinal product management information printed in an optical code, and medicinal product management information encoded in a radio frequency identification (RFID) tag have been integrated, is used.

## Description

### Technical Field

The present invention relates to a medicinal product sales management method, a medicinal product sales management system, a computer program therefor, and a medicinal product information sheet. More specifically, the present invention relates to a medicinal product sales management method, a medicinal product sales management system, a computer program therefor, and a medicinal product information sheet, wherein when a medicinal product, for which an order is placed by a pharmacy to a distributor, is delivered to the pharmacy and the inventory is managed, the management is performed through a single medicinal product information sheet in which medicinal product price information printed in numbers, medicinal product management information printed in an optical code, and medicinal product management information encoded in a radio frequency identification (RFID) tag have been integrated.

### Background Art

Many types of medicinal products are sold in a pharmacy, including prescription medicinal products and over-the-counter medicinal products. In such a pharmacy equipped with various types of medicinal products, efficient inventory management for medicinal products is required to reduce the inventory management burden on a pharmacist.

In general, the pharmacy orders and receives necessary medicinal products through a medicinal product distributor (wholesale store).

To this end, in the pharmacy, inventory management such as ordering, receiving, inspecting, displaying, selling, and returning for medicinal products is performed on a daily or several day basis. In the inventory management process, some medicinal products are not sold, so that they are discarded or returned to a pharmaceutical company (or a medicinal product distributor) after the expiration date thereof has expired, and thus deducted from the inventory.

As an example of medicinal product inventory management in the related art, there is Korean Patent Registration No. 10-1204647, titled "medicinal product inventory management device and method using medicinal product identification information".

The medicinal product inventory management device according to the related art is configured to include an interface that searches a database for order information associated with the medicinal product inventory server, in association with the medicinal product identification information input from the medicinal product inventory server, and a control processor that, when identifying the input medicinal product identification information from the found order information, provides the order information to the medicinal product inventory server to allow medicinal product inventory information to be updated in the medicinal product inventory server, thereby enabling easy management of the medicinal product inventory information.

However, there is a problem that management is only made for the inventory of the medicinal product, and management is not achieved at all for the expiration date of the medicinal product, in the related art.

Therefore, as there are medicinal products that can no longer be sold because the expiration date thereof has expired in each pharmacy, a large loss can occur economically. To prevent this, the pharmacist should check the expiration date of numerous medicinal products every day, which results in a problem of increasing the work load of the pharmacist.

In addition, even when the pharmacist wants to return the expired medicinal product in the related art, distributor information that supplied the corresponding medicinal product is not managed on a medicinal product basis, whereby there is a problem in that the return of the medicine should be processed manually by the pharmacist.

### Disclosure

### Technical Problem

The present invention is envisaged to solve the above problems described above, and an objective of the present invention is to provide a medicinal product sales management method, a medicinal product sales management system, a computer program therefor, and a medicinal product information sheet, wherein when a medicinal product, for which an order is placed by a pharmacy to a distributor, is delivered to the pharmacy and the inventory is managed, the management is performed through a single medicinal product information sheet in which medicinal product price information printed in numbers, medicinal product management information printed in an optical code, and medicinal product management information encoded in a radio frequency identification (RFID) tag have been integrated.

### Technical Solution

In order to achieve the above object, according to one aspect of the present invention, a medicinal product sales management method, which is executed in a sales management system connected to a pharmacy system and a distributor system via a network is provided, the pharmacy system being provided with a barcode scanner and a radio frequency identification (RFID) reader, and the distributor system being provided with a printer and an RFID encoder, the method including 1) receiving medicinal product management information from the distributor system, the medicinal product management information including medicinal product items, medicinal product prices, medicinal product expiration or use-by date, and distributor identification information; 2) receiving an order request for a medicinal product from the pharmacy system; 3) receiving an order acceptance for the medicinal product, for which the order is requested, from the distributor system; 4) receiving generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and 5) receiving goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

Preferably, the present invention may further include 6) generating or updating inventory information for the pharmacy system on the basis of the goods receipt information, the inventory information being information about medicinal products managed as inventory in the pharmacy system on the basis of a time point when the goods receipt information is provided, and including the medicinal product management information; 7) receiving sales information for a medicinal product, for which sales processing is recognized, from the pharmacy system, the sales information being generated by recognizing the sales processing of the medicinal product information sheet attached to the medicinal product through the barcode scanner in the pharmacy system, and including the medicinal product management information; and 8) updating the inventory information of the pharmacy system on the basis of the sales information.

Preferably, the present invention may further include 9) receiving inventory check information from the pharmacy system, the inventory check information being generated by recognizing the inventory check processing of the medicinal product information sheet attached to the medicinal product through the RFID reader in the pharmacy system, and including the medicinal product management information; 10) checking whether the inventory information of the pharmacy system is consistent with the inventory check information on the basis of a time point when the inventory check information is provided, and providing inconsistency item information between the inventory information and the inventory check information to the pharmacy system when the inventory information and the inventory check information are determined to be inconsistent with each other; 11) receiving a inconsistency correction request for the inconsistency item information from the pharmacy system; and 12) updating the inventory information of the pharmacy system on the basis of the inventory check information, for the inventory check information for which the inconsistency correction request is received.

Preferably, the distributor system may be provided with a barcode scanner and an RFID reader, and the method may further include 109) when it is determined that there is a medicinal product subject to notification of which an expiration date or a use-by date satisfies a predetermined notification condition, among medicinal products for which the inventory information is managed, transmitting information on the medicinal product subject to notification to the pharmacy system; 110) receiving a return request for an inventory of the medicinal product subject to notification from the pharmacy system; 111) transmitting the medicinal product management information of the medicinal product for which the inventory return is requested, to the distributor system that delivered the medicinal product for which the inventory return is requested; 112) receiving delivery information for inventory return of the medicinal product, for which the inventory return is requested, from the pharmacy system; and 113) receiving the return goods receipt information for the medicinal product, for which the inventory return is delivered, from the distributor system, the return goods receipt information being generated by recognizing the medicinal product information sheet attached to the medicinal product returned to the distributor through the barcode scanner or the RFID reader in the distributor system.

Preferably, the pharmacy system may be set to any one of a general sales mode, an inventory management mode, an inventory check mode, and an anti-theft mode, which are recognition modes set to recognize information included in the medicinal product information sheet through the barcode scanner or the RFID reader, in which the sales information is generated on the basis of the step 7) in the general sales mode, the inventory information is generated or updated on the basis of the step 6), in the inventory management mode, the inventory check information is generated on the basis of the step 9) in the inventory check mode, and theft warning information is generated in the anti-theft mode, when medicinal product delivery information, which is generated by recognizing the medicinal product information sheet attached to the medicinal product through the RFID reader in the pharmacy system, is inconsistent with the sales information generated in the step 7) .

Preferably, the pharmacy system may further include a display means, and the method may further include: 201) generating sales-linked relationship information between one medicinal product for which sales information is generated and another medicinal product sold together with the same, using the sales information of the pharmacy system generated on the basis of the step 7), when the number of times in which one medicinal product and another medicinal product are sold to a buyer at the same time is equal to or more than a predetermined number of times for a predetermined period, the sales-linked relationship information being generated in the case that it is determined that a sales-linked relationship exists between the medicinal products; and 202) providing the sales-linked relationship information about medicinal products managed as the inventory in the pharmacy system, when the sales processing is recognized for any one medicinal product on the basis of the step 7), the pharmacy system outputting, through the display means, advertisement information on another medicinal product having the sales-linked relationship with the medicinal product, on the basis of the sales-linked relationship information.

Preferably, in the step 202), when the sales processing is recognized for any one medicinal product on the basis of the step 7), the pharmacy system may be configured to output, through the display means, advertisement information on medicinal products managed as inventory in the pharmacy system among the medicinal products having the sales-linked relationship with the medicinal product.

According to another aspect of the present invention, a sales management system, including a memory storing one or more instructions; and a processor executing the one or more instructions stored in the memory is provided, wherein the processor is configured to receive medicinal product management information from the distributor system, the distributor system being provided with a printer and an RFID encoder and, the medicinal product management information including medicinal product items, medicinal product prices, medicinal product expiration or use-by date, and distributor identification information; receive an order request for a medicinal product from the pharmacy system, the pharmacy system being provided with a barcode scanner and a radio frequency identification (RFID) reader; receive an order acceptance for the medicinal product, for which the order is requested, from the distributor system; receive generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and receive goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

According to another aspect of the present invention, a computer program stored on media to execute a medicinal product sales management method in combination with hardware is provided, the medicinal product sales management method including receiving medicinal product management information from the distributor system, the distributor system being provided with a printer and an RFID encoder and, the medicinal product management information including medicinal product items, medicinal product prices, medicinal product expiration or use-by date, and distributor identification information; receiving an order request for a medicinal product from the pharmacy system, the pharmacy system being provided with a barcode scanner and a radio frequency identification (RFID) reader; receiving an order acceptance for the medicinal product, for which the order is requested, from the distributor system; receiving generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and receiving goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

According to another aspect of the present invention, a medicinal product information sheet formed in such a manner as to be attached to a medicinal product package and including medicinal product management information is provided, the medicinal product management information including a medicinal product item, a medicinal product price, an expiration date or use-by date of a medicinal product, and distributor identification information, wherein the medicinal product information sheet is configured so that a first information element in which medicinal product price information among the medicinal product management information is printed in numbers, a second information element in which the medicinal product management information is printed in an optical code, and a third information element in which the medicinal product management information is encoded in an RFID tag are included in one sheet.

### Advantageous Effects

According to the present invention, there is an advantage in that when a medicinal product, for which an order is placed by a pharmacy to a distributor, is delivered to the pharmacy and the inventory is managed, the management can be performed through a single medicinal product information sheet in which medicinal product price information printed in numbers, medicinal product management information printed in an optical code, and medicinal product management information encoded in a radio frequency identification (RFID) tag have been integrated, thereby achieving management convenience.

In addition, according to the present invention, there are advantages that the management for inventory of medicinal products and the management for the expiration date of medicinal products can be performed, and the management for information on a distributor supplying the drug can be performed as well to conveniently carry out the returns of medicinal products of which the expiration date has expired.

### Description of Drawings

FIG. 1 is an overall configuration view showing a system in which a medicinal product sales management method according to an embodiment of the present invention is executed.
FIG. 2 is a configuration view showing a sales management system according to an embodiment of the present invention.
FIG. 3 is a configuration view showing a pharmacy system according to an embodiment of the present invention.
FIG. 4 is a configuration view showing a distributor system according to an embodiment of the present invention.
FIGS. 5 to 9 are flowcharts showing a medicinal product sales management method according to an embodiment of the present invention.
FIG. 10 is an exemplary view showing a medicinal product information sheet according to an embodiment of the present invention.
FIG. 11 is a schematic diagram showing a sales management system from a hardware perspective according to an embodiment of the present invention.

### Mode for Invention

The present invention can be implemented in various other forms without departing from its technical spirit or main characteristics. Therefore, the embodiments of the present invention are merely illustrative in all respects and should not be interpreted as limiting.

Terms such as first and second are used only for the purpose of distinguishing one component from other components. For example, the first component may be referred to as a second component without departing from the scope of the present invention, and similarly, the second component may be referred to as a first component.

When an element is said to be "connected" or "coupled" to other component, the element may be directly connected to or connected to the other component, but another components may exist in between.

The singular expression used in the present application includes the plural expression unless the context clearly indicates otherwise. In the present application, the terms "comprise", "have", or "include", etc. are intended to express the existence of elements or combinations thereof described in the specification, and do not preclude the possibility that other elements or features are present or added.

Hereinafter, preferred embodiments of the present invention will be described in detail with reference to the accompanying drawings.

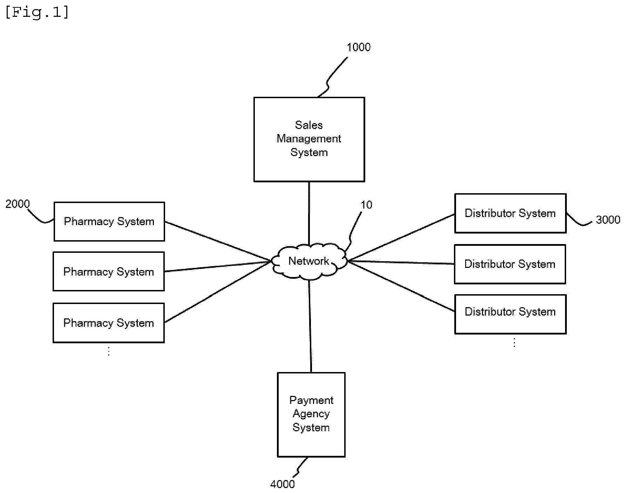

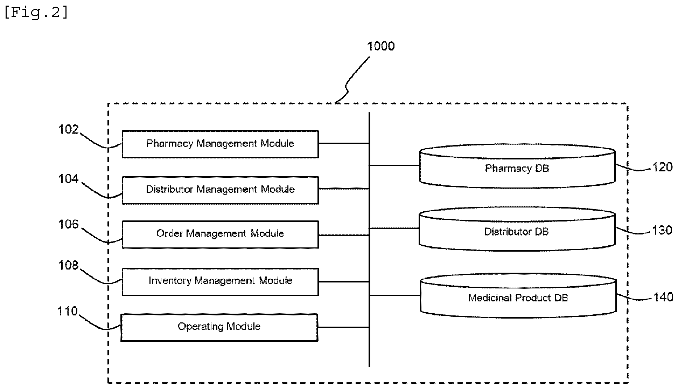

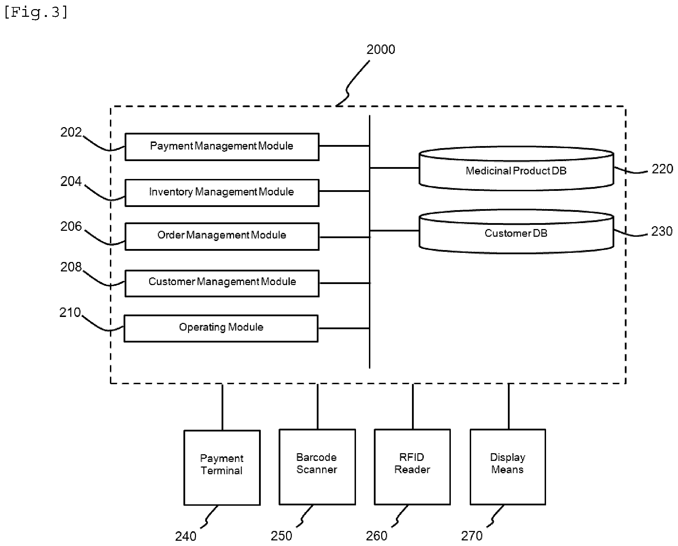

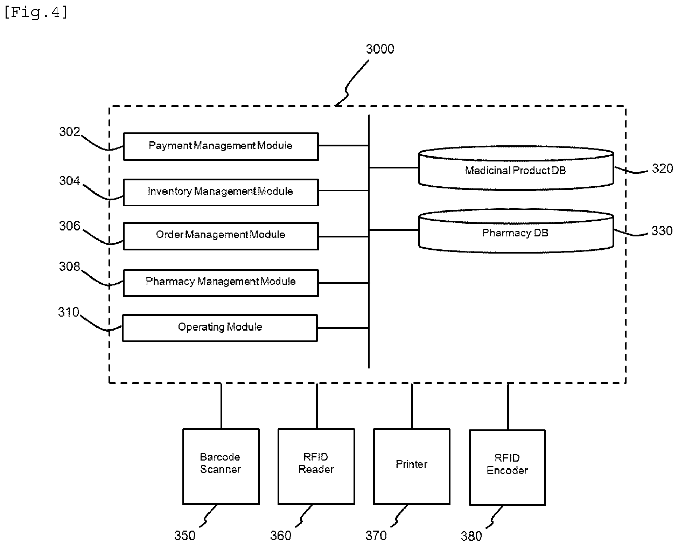

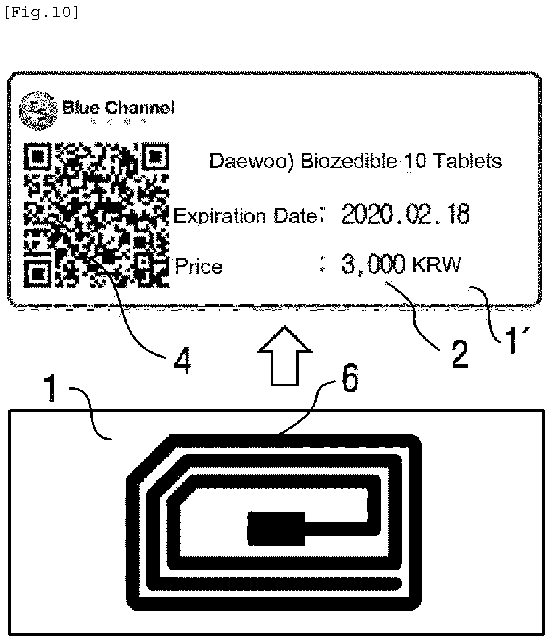

FIG. 1 is an overall configuration view showing a system in which a medicinal product sales management method according to an embodiment of the present invention is executed; FIG. 2 is a configuration view showing a sales management system according to an embodiment of the present invention; FIG. 3 is a configuration view showing a pharmacy system according to an embodiment of the present invention; FIG. 4 is a configuration view showing a distributor system according to an embodiment of the present invention; and FIG. 10 is an exemplary view showing a medicinal product information sheet according to an embodiment of the present invention.

The medicinal product sales management method according to the present embodiment is executed using a medicinal product information sheet, which is formed in such a manner as to be capable of being attached to the medicinal product packaging as illustrated in FIG. 10 and includes medicinal product management information. The medicinal product management information includes a medicinal product item, a medicinal product price, expiration date or use-by date of medicinal product, and distributor identification information.

The medicinal product information sheet according to this embodiment is configured so that a first information element 2 in which medicinal product price information among the medicinal product management information is printed in numbers on the sheet surface, a second information element 4 in which the medicinal product management information is printed in an optical code on the sheet surface, and a third information element 6 in which the medicinal product management information is encoded in an RFID tag attached to a sheet are included in one sheet 1, 1'.

As an example, the optical code is formed in a known two dimensional optical code, such as a QR (Quick Response) code. Unlike one dimensional optical code, such as barcode, the two dimensional optical code uses a matrix type code pattern and is capable of recording a large amount of information. For QR codes, up to 7,089 numbers, up to 4,296 characters (ASCII), up to 2,953 bytes in binary (8 bits), and up to 1,817 Chinese characters may be stored therein.

For example, the RFID tag may be included in the medicinal product information sheet in such a manner that the RFID tag is attached to or embedded in a paper or synthetic resin sheet 1' on which the first information element 2 and the second information element 4 are printed. The two sheets 1, 1' for attaching tags may be adhered to each other, for RFID tag attachment or embedment configuration.

As another example, the medicinal product price information and the medicinal product sales management information in the form of optical code may be printed on the sheet surface using a printer, in a state that the RFID tag is attached to or embedded in a paper or synthetic resin sheet. When printing on the sheet to which an RFID tag is attached or embedded, it is preferable to use an inkjet printer with low heat generation in order to prevent damage to the RFID chip due to heat.

The RFID tag may be used with, for example, a passive RFID tag, and formed in an appropriate pattern structure according to used frequency conditions.

The passive RFID tag is a type without a battery that supplies power to the tag, in which the tag power source depends on and responds to energy of a RFID reader. In the case of the pharmacy system according to this embodiment, the pharmacy system is used in an indoor environment and thus it does not matter even when the recognition distance is relatively short, and the RFID tags must be attached to various medicinal products and thus it is better to use relatively low-cost RFID tags. In view of this, it is preferable to use the passive RFID tag.

As an example, the RFID tag and the RFID reader according to the present embodiment is preferably used in a microwave frequency band of 860 ∼ 960 MHz. Since the passive RFID in the microwave frequency band has a recognition distance of about 10m or less, there are advantages that the passive RFID is suitable for use in a pharmacy indoor environment and is capable of implementation in a low cost.

A sales management system 1000 according to the present embodiment is connected to one or more pharmacy system 2000 and one or more distributor systems 3000, a payment agency system 4000 through a network 10, and implements a medicinal product sales management method, including medicinal product supply to pharmacies, medicinal product order for distributors, payment for pharmacies and distributors, inventory management, return process, and the like.

As an example, the "sales management system 1000" according to the present embodiment may be understood as a sales manager system configured to receive orders for medicinal products from one or more pharmacy systems 2000, process the ordered medicinal products with one or more distributor systems 3000, and process the supply of the medicinal products to an individual pharmacy through a distributor.

In particular, the sales management system 1000 according to the present embodiment is connected to a pharmacy system 2000 having a payment terminal 240, a barcode scanner 250, an RFID reader 260, and a display means 270 via a network and is configured to manage and provide information on order management, goods receipt management, inventory management, inventory return management for medicinal products in each pharmacy system 2000.

The pharmacy system 2000 is a medicinal product management system operated by individual pharmacy, and is provided with a payment terminal 240 for payment processing of the customer who purchased the medicinal product, and a barcode scanner 250 and an RFID reader 260 for medicinal product recognition. The pharmacy system 200 is connected to the sales management system 1000 and the payment agency system 4000 via a network 10 to perform overall functions of the medicinal product sales management in the pharmacy, including medicinal product order, inventory management, customer payment processing, headquarters payment processing, return processing, and the like.

Preferably, the pharmacy system according to this embodiment is configured so that the barcode scanner 250 suitable for individually recognizing medicinal products is used to ensure accurate sales processing when performing sales processing of medicinal products, and the efficiency of inventory management is promoted by using the RFID reader 260 capable of simultaneously recognizing multiple medicinal products when checking the inventory of medicinal products stored in pharmacy.

The pharmacy system 2000 according to this embodiment is provided with a display means 270. Advertisement information on another medicinal product having a sales-linked relationship with medicinal products processed for sale, which is to be described later, may be output through the display means 270.

The pharmacy system 2000 according to this embodiment may obtain medicinal product purchase information regarding a medicinal product purchase item of a customer using the payment terminal 240 and the barcode scanner 250.

In addition, the pharmacy system 2000 according to the present embodiment may obtain the medicinal product inventory management information using the RFID reader 260.

The distributor system 3000 according to this embodiment may be understood as a system of a manufacturer or distributor, which receives orders from the sales management system 1000 and supplies medicinal products to an individual pharmacy.

The payment agency system 4000 according to this embodiment can be understood as a system, such as a credit card company, a bank, and the like, which processes the payment for medicinal product price or the sales management fee among the pharmacy system 2000, the sales management system 1000, and the distributor system 3000, with regard to sales of medicinal products between customers and the pharmacy system 2000, and ordering, goods receipt, and returning of medicinal products between the pharmacy system 2000 and distributor system 3000.

The network 10 according to the present embodiment means a common communication network such as the Internet or an intranet, and may be understood to encompass both wired and wireless networks.

Meanwhile, in the above description, "system" refers to a system of a logical concept that serves as a server or a client in a typical server-client environment, and may be implemented through, for example, general computing means (e.g., PC, tablet PC, smartphone, and server computer) capable of using web services or app services. The systems according to this embodiment may have an input console such as a keyboard and a mouse, and may also include display means such as a monitor and voice output means such as a speaker.

Meanwhile, the sales management system 1000 of the present embodiment includes a pharmacy management module 102 for registration and management of pharmacies, a distributor management module 104 for registering and managing distributors, an order management module 106 for medicinal product order processing of pharmacies and distributors, an inventory management module 108 for inventory management and return processing of pharmacies and distributors, and an operating module 112 for general system environment operation.

In addition, the sales management system 1000 according to the present embodiment includes a pharmacy DB 120 that manages pharmacy-related information, a distributor DB 130 that manages distributor-related information, and a medicinal product DB 140 that manages information related to supply, inventory, and returns of medicinal products.

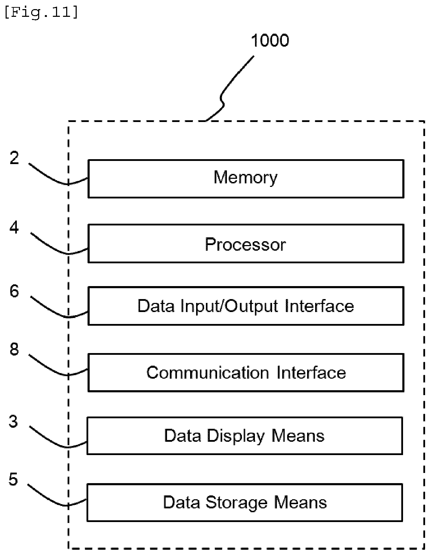

Referring to FIG. 11, from a hardware perspective, the sales management system 1000 according to this embodiment includes a memory for storing one or more instructions 2 and a processor 4 executing one or more instructions stored in the memory 2 and is a computing device in which a computer program stored on a medium is executed to execute the medicinal product sales management method. The sales management system 1000 according to the present embodiment may include a data input/output interface 6, a communication interface 8, data display means 3, and data storage means 5.

The pharmacy system 2000 according to this embodiment includes a payment terminal 240 for payment processing of customers who have purchased medicinal products, and the barcode scanner 250 and RFID reader 260 for recognition of the medicinal product management information, and further includes a display means 270 for outputting advertisement information for a medicinal product. As an example, the advertisement information for the medicinal product may be received from the sales management system 1000 and provided to the display means in a streaming method or a stored file playback method. The payment terminal 240 is a typical terminal that receives the medicinal product information to be sold, calculates the payment amount, and executes payment processing in a local or network type by reading a credit card, entering a cash amount, and so on.

The pharmacy system 2000 according to this embodiment includes a payment management module 202 for customer payment processing and distributor payment processing, an inventory management module 204 for medicinal product inventory information management, an order management module 206 for medicinal products order and return processing for the sales management system, a customer management module 208 for customer information obtainment and management, and operating module 210 for general system environment operation.

In addition, the pharmacy system 2000 according to the present embodiment includes a medicinal product DB 220 that manages medicinal product-related information and a customer DB 230 that manages customer-related information.

The module configuration of each system described above is a functional concept, and of course, some modules may be integrated into one module or separately configured into detailed functions.

The distributor system 3000 according to this embodiment includes a barcode scanner 350 and RFID reader 360 for recognition of the medicinal product management information, a printer 370 for printing the first information element 2 and the second information element 4 on the medicinal product information sheet 1, 1', and an RFID encoder 380 for encoding the third information element 6 on an RFID tag attached to a sheet. The printer 370 and the RFID encoder 380 may be provided in the form of individual devices, and be provided in the form of an integrated device. The distributor system 3000 according to the present embodiment may include a payment terminal (not shown) for inputting medicinal product information delivered to pharmacy.

The distributor system 3000 according to this embodiment includes a payment management module 302 for pharmacy payment processing and sales manager payment processing, an inventory management module 304 for managing inventory information for each pharmacy or each medicinal product, an order management module 306 for medicinal products order and return processing for pharmacy systems, a pharmacy management module 308 for pharmacy information management, and an operating module 310 for general system environment operation.

In addition, the distributor system 3000 according to this embodiment includes a medicinal product DB 320 for managing medicinal product-related information and a pharmacy DB 330 that manages pharmacy-related information.

The module configuration of each system described above is a functional concept, and of course, some modules may be integrated into one module or separately configured into detailed functions.

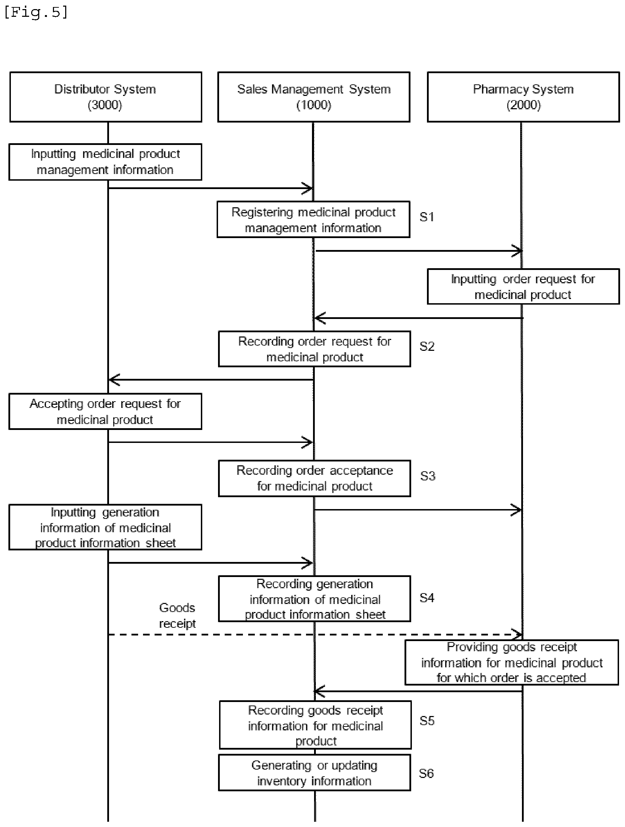

With reference to FIGS. 5 to 9, a process of executing a medicinal product sales management method according to an embodiment of the present invention will be described.

FIG. 5 illustrates a process in which a medicinal product information sheet is generated as medicinal product management information is input and then goods receipt in a pharmacy is processed, in the medicinal product sales management method according to the present embodiment.

The medicinal product sales management method according to the present embodiment is executed in a sales management system 1000 which is connected to the pharmacy system 2000 and the distributor system 3000 via the network 10.

The pharmacy system 2000 includes a barcode scanner 250 and a radio frequency identification (RFID) reader 260, and the distributor system 3000 includes a printer 370 and an RFID encoder 380.

In step S1, the sales management system 1000 registers medicinal product management information from the distributor system 3000. The medicinal product management information includes a medicinal product item, a medicinal product price, expiration date or use-by date of medicinal product, and distributor identification information. For example, information for medicinal product management information registration may be input through an input console of the distributor system 3000.

For example, the distributor identification information may be input when inputting individual medicinal product management information through the input console of the distributor system 3000. As another example, when the distributor system 3000 logs in to the sales management system 1000, the distributor identification information may be automatically recognized. Through the input of the distributor identification information, the convenience of the return procedure may be improved during inventory return management, which will be described later.

In step S2, the sales management system 1000 receives an order request for a medicinal product from the pharmacy system 2000. For example, the medicinal product order request may be input through an input console of the pharmacy system 2000. The medicinal product order request may include medicinal product item information, quantity information, and distributor information to which the order is requested. As another example, the distributor is not specified, and only medicinal product item information and quantity information may be included in the medicinal product order request.

In step S3, the sales management system 1000 receives an order acceptance for the medicinal product, for which the order is requested, from the distributor system 3000. To this end, the sales management system 1000 may provide the medicinal product order request to the distributor system 3000 of the distributor included in the order request. When a specific distributor is not specified in the medicinal product order request, the medicinal product order request may be provided to two or more distributor systems 3000 that keep in stock the medicinal product for which the order is requested.

In step S4, the sales management system 1000 receives generation information of the medicinal product information sheet of the medicinal product, for which the order is accepted, from the distributor system 3000.

In step S3, when the medicinal product order request is provided to 2 or more distributor systems 3000, the generation information of the medicinal product information sheet is received from one distributor system 3000 selected according to a preset criterion (e.g., acceptance order).

The generation information of the medicinal product information sheet is, for example, information that checks the output and generation of the medicinal product information sheet of the medicinal products, for which the order is accepted, using a printer and an RFID encoder. Through this, the sales management system 1000 may record and manage which medicinal product information sheet is created for which medical products and for which distributors.

The medicinal product information sheet is configured so that a first information element in which medicinal product price information among the medicinal product management information is printed in numbers, a second information element in which the medicinal product management information is printed in an optical code, and a third information element in which the medicinal product management information is encoded in an RFID tag are included in one sheet.

In step S5, the sales management system 1000 receives goods receipt information for the medicinal product, for which the order is received, from the pharmacy system 2000.

The goods receipt information is generated by recognizing information contained in the medicinal product information sheet attached to the medicinal product received in the pharmacy through the barcode scanner 250 or the RFID reader 260 in the pharmacy system 2000 and contains the medicinal product management information. The goods receipt information may include information on the pharmacy in which the medicinal product is received.

For example, the pharmacy information may be automatically input when the pharmacy system 2000 recognizes the medicinal product information sheet. As another example, the identification information may be automatically recognized when logging in to the sales management system 1000 in the pharmacy system 2000.

The goods receipt information is recorded and managed in the sales management system 1000.

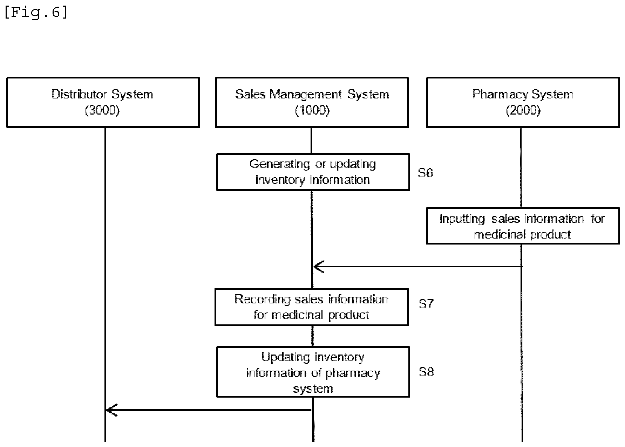

FIG. 6 illustrates a process of managing inventory information for each pharmacy according to medicinal product sales, in medicinal product sales management methods according to this embodiment.

In step S6, the sales management system 1000 generates or updates the inventory information for the pharmacy system 2000 on the basis of the goods receipt information. The creation or update of inventory information is performed on an individual pharmacy basis.

The inventory information is information on medicinal products managed as inventory in the individual pharmacy system 2000 on the basis of the time point when the provision of the goods receipt information is completed, and contains the medicinal product management information.

In step S7, the sales management system 1000 receives sales information for a medicinal product, for which the sales processing is recognized, from the pharmacy system 2000.

The sales information is generated by recognizing the medicinal product information sheet attached to the medicinal product through the barcode scanner 250 in the pharmacy system 2000, and includes the medicinal product management information. According to this embodiment, when selling the medicinal products, the medicinal products may be accurately sold using a barcode scanner 250 suitable for recognition of individual medicinal product.

In step S8, the sales management system 1000 updates inventory information of the pharmacy system 2000 on the basis of the sales information.

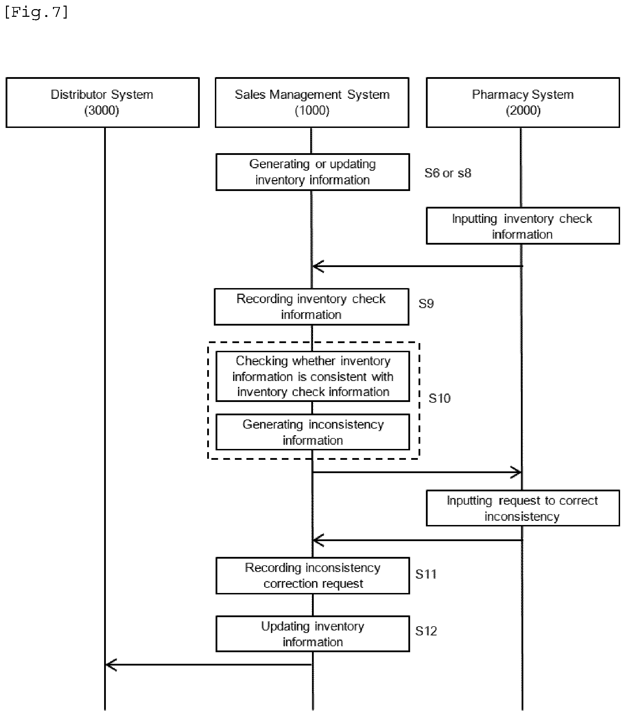

FIG. 7 is an exemplary view showing a process for checking inventory for each pharmacy in the medicinal product sales management method of the present embodiment.

In step S9, the sales management system 1000 receives inventory check information from the pharmacy system 2000.

The inventory check information is generated by recognizing the inventory check processing of the medicinal product information sheet attached to the medicinal product through the RFID reader 260 in the pharmacy system 2000, and contains the medicinal product management information. According to this embodiment, when checking the inventory of medicinal products stored in a pharmacy, it is possible to promote the efficiency of inventory management using RFID reader 260 capable of recognizing multiple medicinal products at the same time.

In step S10, the sales management system 1000 checks whether the inventory information of the pharmacy system 2000 is consistent with the inventory check information on the basis of the time point when the inventory check information is provided. When it is determined that the inventory information and the inventory check information are inconsistent with each other, inconsistency item information between inventory information and inventory check information is provided to the pharmacy system 2000. Checking whether the inventory information and the inventory check information are consistent with each other may be performed by checking data on medicinal product items, quantities, and expiration date/use-by date.

In step S11, the sales management system 1000 receives a inconsistency correction request regarding the inconsistency item information from the pharmacy system 2000.

In step S12, in the case of inventory check information for which the inconsistency correction request has been received, the sales management system 1000 updates inventory information of the pharmacy system 2000 on the basis of the inventory check information.

Meanwhile, the pharmacy system 2000 may be set to any one of a general sales mode, an inventory management mode, an inventory check mode, and an anti-theft mode, which are recognition modes set to recognize information included in the medicinal product information sheet through the barcode scanner 250 or the RFID reader 260. Such mode setting may be performed through an input console of the pharmacy system 2000. The mode setting makes it possible to perform input control, such as priority setting and limit setting of information recognition, through the barcode scanner 250 or the RFID reader 260.

In the general sales mode, the sales information is generated on the basis of step S7.

In the inventory management mode, the inventory information is generated or updated on the basis of step S6.

In the inventory check mode, the inventory check information is generated on the basis of step S9. To prevent input errors or input duplication in inventory check mode, the pharmacy system 2000 may make input inactive through the barcode scanner 250 and make input active through the RFID reader 260.

In the anti-theft mode, when medicinal product delivery information, which is generated by recognizing the medicinal product information sheet attached to the medicinal product through the RFID reader 260 in the pharmacy system 2000, is inconsistent with the sales information generated in step S7, theft warning information is generated.

In the anti-theft mode, the pharmacy system 2000 activates all inputs through the barcode scanner 250 and the RFID reader 260. When the sales payment is first processed through the barcode scanner 250, and then the medicinal product delivery is recognized by the RFID reader 260 for one medicinal product, it is recognized as a normal delivery, not theft.

To this end, the RFID reader 260 of the pharmacy system 2000 is installed in a side of an exit of the pharmacy to recognize the medicinal product information sheet of medicinal products that are taken out of the pharmacy through the exit.

That is, when the medicinal product delivery information generated by recognizing the medicinal product information sheet attached to the medicinal product through the RFID reader 260 in the pharmacy system 2000 is consistent with the sales information generated in step S7, it is determined that the medicinal product that has been processed as normal payment is taken out of the pharmacy and thus the medicinal product is determined not to be stolen. On the contrary, when they are consistent with each other, it is determined that the medicinal product that has not been processed as normal payment is taken out of the pharmacy, thereby generating theft warning information. The theft warning may be output through video or audio output means (not shown).

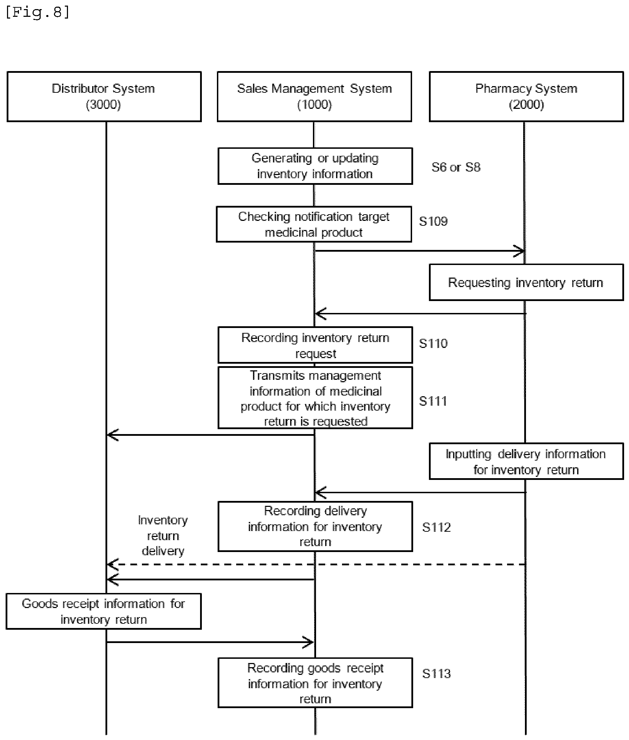

FIG. 8 is an exemplary view showing a notification processing process and an inventory return process as an expiration date/use-by date has expired, in the medicinal product sales management method according to this embodiment.

The distributor system 3000 includes a barcode scanner 250 and an RFID reader 260.

In step S109, when it is determined that there is a medicinal product subject to notification (notification target medicinal product) of which an expiration date or a use-by date satisfies a predetermined notification condition(e.g., within one month of the expiration date), among medicinal products for which inventory information is managed, the sales management system 1000 transmits information about the medicinal product subject to notification to the pharmacy system 2000 that stores the medicinal product subject to notification as inventory.

In step S110, the sales management system 1000 receives an inventory return request for the medicinal product subject to notification from the pharmacy system 2000.

In step Sill, the sales management system 1000 transmits medicinal product management information of the medicinal product, for which the inventory return is requested, to the distributor system 3000 delivering the medicinal product, for which the inventory return is requested. As described above, since the distributor identification information is included in the medicinal product management information, there is an advantage that when managing the inventory return, it is possible to identify the distributor that supplied the medicinal product and then make a return request for the inventory.

In step S112, the sales management system 1000 is provided with inventory return delivery information regarding a medicinal product, for which the inventory return is requested, from the pharmacy system 2000. The input of inventory return delivery information may be recognized by the pharmacy system 2000 through the barcode scanner 250 or the RFID reader 260, or may be input in bulk via a console input in the case of bulk packaging.

In step S113, the sales management system 1000 receives return goods receipt information for the medicinal product, which is delivered as the inventory return, from the distributor system 3000.

The return goods receipt information is generated by recognizing the medicinal product information sheet attached to the medicinal product returned to the distributor through the barcode scanner 250 or the RFID reader 260 in the distributor system 3000.

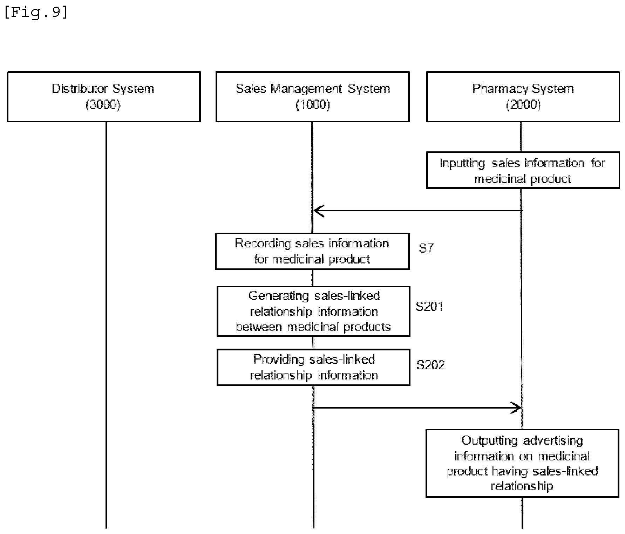

FIG. 9 is an exemplary view showing a process of generating information on a sales-linked relationship between medicinal products and performing a medicinal product advertisement in a pharmacy using the same, in the medicinal product sales management methods of the present embodiment.

The pharmacy system 2000 is provided with a display means.

In step S201, the sales management system 1000 generates sales-linked relationship information between the medicinal products, for which sales information is generated, and another medicinal product sold together with the same, using the sales information of the pharmacy system 2000 generated in step S7.

When the number of times in which one medicinal product and another medicinal product are sold to a buyer at the same time is equal to or more than a predetermined number of times for a predetermined period, it is determined that a sales-linked relationship exists between the medicinal products, and sales-linked relationship information is generated between the medicinal products.

For example, in the case of a male customer in his 40s, when he tends to buy a specified digestive medicine and a nutrition product, other male customers in their 40s are likely to have tendencies to purchase similar medicinal products according to similar health conditions. In this case, for male customers in their 40s, it may be determined that the specified digestive medicine and the nutrition product have a sales-linked relationship with each other.

By utilizing such a sales-linked relationship, when providing guide information or advertising information about a specific nutrition product having a sales-linked relationship with a specified digestive medicine, to male customers in their 40s who purchase the specified digestive medicine, at a time point when purchasing the specific digestive medicine, the pharmacy may provide beneficial nutrition product information to the customer and the customer also may obtain information about the nutrition products which are beneficial to him and it causes purchasing factors.

In step S202, the sales management system 1000 provides sales-linked relationship information about a medicinal product managed as inventory in the pharmacy system 2000.

On the basis of the sales-linked relationship information, when the sales processing is recognized for any one medicinal product in the step S7, the pharmacy system 2000 is configured so that advertisement information on another medicinal product having a sales-linked relationship with the medicinal product is displayed via a display means.

Preferably, upon sales processing is recognized for any one medicinal product in the step S7, the pharmacy system 2000 is configured so that advertisement information on medicinal products managed as the inventory in the pharmacy system 2000 is displayed via a display means, among other medicinal products having sales-linked relationship with the medicinal product, in the step S202.

The pharmacy system 2000 may receive information on medicinal products managed as inventory from the sales management system 1000.

By utilizing the sales-linked relationship, when the pharmacy system provides beneficial medicinal product guidance information to the customer, particularly, information about medicinal products that are managed as inventory in the pharmacy, there is an advantage of being capable of inducing sales for the medicinal product.

Meanwhile, embodiments of the present invention include program instructions for performing various computer-implemented operations and computer-readable media including the same. The computer-readable medium may include program instructions, data files, data structures, or the like alone or in combination. The media may be specially designed and constructed for the present invention, or may be known and usable to those skilled in computer software. Examples of computer-readable recording media includes magnetic media, such as hard disks, floppy disks, and magnetic tapes, optical recording media such as CD-ROM, DVD, USB drives, magnetic-optical media such as floptical discs, and hardware device specially configured to store and execute program instructions such as ROM, RAM, flash memory, etc. Examples of program commands includes machine code like one produced by the compiler, as well as high-level language code that can be executed by a computer using an interpreter, etc.

## Claims

1. A medicinal product sales management method, which is executed in a sales management system connected to a pharmacy system and a distributor system via a network, the pharmacy system being provided with a barcode scanner and a radio frequency identification (RFID) reader, and the distributor system being provided with a printer and an RFID encoder, the method comprising:
1) receiving medicinal product management information from the distributor system, the medicinal product management information including medicinal product items, medicinal product prices, medicinal product expiration or use-by date, and distributor identification information;
2) receiving an order request for a medicinal product from the pharmacy system;
3) receiving an order acceptance for the medicinal product, for which the order is requested, from the distributor system;
4) receiving generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and
5) receiving goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

2. The method of claim 1, further comprising:
6) generating or updating inventory information for the pharmacy system on the basis of the goods receipt information, the inventory information being information about medicinal products managed as inventory in the pharmacy system on the basis of a time point when the goods receipt information is provided, and including the medicinal product management information;
7) receiving sales information for a medicinal product, for which sales processing is recognized, from the pharmacy system, the sales information being generated by recognizing the sales processing of the medicinal product information sheet attached to the medicinal product through the barcode scanner in the pharmacy system, and including the medicinal product management information; and
8) updating the inventory information of the pharmacy system on the basis of the sales information.

3. The method of claim 2, further comprising:
9) receiving inventory check information from the pharmacy system, the inventory check information being generated by recognizing the inventory check processing of the medicinal product information sheet attached to the medicinal product through the RFID reader in the pharmacy system, and including the medicinal product management information;
10) checking whether the inventory information of the pharmacy system is consistent with the inventory check information on the basis of a time point when the inventory check information is provided, and providing inconsistency item information between the inventory information and the inventory check information to the pharmacy system when the inventory information and the inventory check information are determined to be inconsistent with each other;
11) receiving a inconsistency correction request for the inconsistency item information from the pharmacy system; and
12) updating the inventory information of the pharmacy system on the basis of the inventory check information, for the inventory check information for which the inconsistency correction request is received.

4. The method of claim 2, wherein the distributor system is provided with a barcode scanner and an RFID reader,
109) when it is determined that there is a medicinal product subject to notification of which an expiration date or a use-by date satisfies a predetermined notification condition, among medicinal products for which the inventory information is managed, transmitting information on the medicinal product subject to notification to the pharmacy system;
110) receiving a return request for an inventory of the medicinal product subject to notification from the pharmacy system;
111) transmitting the medicinal product management information of the medicinal product for which the inventory return is requested, to the distributor system that delivered the medicinal product for which the inventory return is requested;
112) receiving delivery information for inventory return of the medicinal product, for which the inventory return is requested, from the pharmacy system; and
113) receiving the return goods receipt information for the medicinal product, for which the inventory return is delivered, from the distributor system, the return goods receipt information being generated by recognizing the medicinal product information sheet attached to the medicinal product returned to the distributor through the barcode scanner or the RFID reader in the distributor system.

5. The method of claim 3, wherein the pharmacy system is set to any one of a general sales mode, an inventory management mode, an inventory check mode, and an anti-theft mode, which are recognition modes set to recognize information included in the medicinal product information sheet through the barcode scanner or the RFID reader,
in which the sales information is generated on the basis of the step 7) in the general sales mode,
the inventory information is generated or updated on the basis of the step 6), in the inventory management mode,
the inventory check information is generated on the basis of the step 9) in the inventory check mode, and
theft warning information is generated in the anti-theft mode, when medicinal product delivery information, which is generated by recognizing the medicinal product information sheet attached to the medicinal product through the RFID reader in the pharmacy system, is inconsistent with the sales information generated in the step 7).

6. The method of claim 2, wherein the pharmacy system further includes a display means,
further comprising:
201) generating sales-linked relationship information between one medicinal product for which sales information is generated and another medicinal product sold together with the same, using the sales information of the pharmacy system generated on the basis of the step 7), when the number of times in which one medicinal product and another medicinal product are sold to a buyer at the same time is equal to or more than a predetermined number of times for a predetermined period, the sales-linked relationship information being generated in the case that it is determined that a sales-linked relationship exists between the medicinal products; and
202) providing the sales-linked relationship information about medicinal products managed as the inventory in the pharmacy system, when the sales processing is recognized for any one medicinal product on the basis of the step 7), the pharmacy system outputting, through the display means, advertisement information on another medicinal product having the sales-linked relationship with the medicinal product, on the basis of the sales-linked relationship information.

7. The method of claim 6, wherein in the step 202), when the sales processing is recognized for any one medicinal product on the basis of the step 7), the pharmacy system is configured to output, through the display means, advertisement information on medicinal products managed as inventory in the pharmacy system among the medicinal products having the sales-linked relationship with the medicinal product.

8. A sales management system, comprising a memory storing one or more instructions; and a processor executing the one or more instructions stored in the memory,
wherein the processor is configured to
receive medicinal product management information from the distributor system, the distributor system being provided with a printer and an RFID encoder and, the medicinal product management information including medicinal product items, medicinal product prices, medicinal product expiration or use-by date, and distributor identification information;
receive an order request for a medicinal product from the pharmacy system, the pharmacy system being provided with a barcode scanner and a radio frequency identification (RFID) reader;
receive an order acceptance for the medicinal product, for which the order is requested, from the distributor system;
receive generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and
receive goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

9. A computer program stored on media to execute a medicinal product sales management method in combination with hardware, the medicinal product sales management method comprising:
receiving medicinal product management information from the distributor system, the distributor system being provided with a printer and an RFID encoder and, the medicinal product management information including medicinal product items, medicinal product prices, medicinal product expiration or use-by date, and distributor identification information;
receiving an order request for a medicinal product from the pharmacy system, the pharmacy system being provided with a barcode scanner and a radio frequency identification (RFID) reader;
receiving an order acceptance for the medicinal product, for which the order is requested, from the distributor system;
receiving generation information of a medicinal product information sheet of the medicinal product, for which the order is accepted, from the distributor system, the medicinal product information sheet being configured so that a first information element in which medicinal product price information among the medicinal product management information is printed in numbers, a second information element in which the medicinal product management information is printed in an optical code, and a third information element in which the medicinal product management information is encoded in the RFID tag are included in one sheet; and
receiving goods receipt information for the medicinal product, for which the order is accepted, from the pharmacy system, the goods receipt information being generated by recognizing information included in the medicinal product information sheet attached to a medicinal product received in a pharmacy through the barcode scanner or an RFID reader in the pharmacy system, and including the medicinal product management information.

10. A medicinal product information sheet formed in such a manner as to be attached to a medicinal product package and including medicinal product management information, the medicinal product management information including a medicinal product item, a medicinal product price, an expiration date or use-by date of a medicinal product, and distributor identification information,
wherein the medicinal product information sheet is configured so that a first information element in which medicinal product price information among the medicinal product management information is printed in numbers, a second information element in which the medicinal product management information is printed in an optical code, and a third information element in which the medicinal product management information is encoded in an RFID tag are included in one sheet.
